# EUROPEAN PATENT APPLICATION

(11) **EP 1 961 310 A1**
(43) Date of publication of application: **27.08.2008**
(21) Application number: 07002144.9
(22) Date of filing: 01.02.2007
(51) Int. Cl.: A23L 1/30, A61K 31/355

(54) **Novel use of (-) -epigallocatechin gallate**

(71) Applicant: DSM IP Assets B.V., 6411 TE Heerlen (NL)
(72) Inventor: The designation of the inventor has not yet been filed
(74) Representative: Rabanus, Birgit

(57) **Abstract**

Use of (-)-epigallocatechin gallate and/or one or more of its derivatives for increasing the desire, willingness and/or motivation of animals (including humans), especially mammals, to exercise, to perform and/or to run, especially to perform endurance exercise.

## Description

The present invention relates to novel uses of (-)-epigallocatechin gallate (EGCG) and dietary supplements, nutraceutical compositions, food, feed and beverages containing (-)-epigallocatechin gallate.

The compositions of the present invention are particularly intended for increasing the desire, willingness and/or motivation of animals (including humans), especially mammals, to exercise, to perform and/or to run, especially to perform endurance exercise.

The term "EGCG" as used herein denotes (-)-epigallocatechin gallate and/or one or more of its derivatives (e. g. esterified forms, glycosides, sulphates) thereof. Especially preferred is (-)-epigallocatechin gallate itself.

The terms "(-)-epigallocatechin gallate" and "EGCG" as used herein encompass naturally occurring sources (one ore more components) containing EGCG, for example green tea and/or green tea extracts, e.g. aqueous green tea extracts containing EGCG and the like. According to the present invention. it may also be advantageous to use synthetically manufactured EGCG.

In preferred embodiments of the present invention the used EGCG has a high purity, e.g. a purity of at least 80 %, preferably of at least 85 %, more preferably of at least 90 %, even more preferably of at least 92 %, most preferably of at least 94 %.

The term "nutraceutical" as used herein denotes usefulness in both, the nutritional and pharmaceutical field of application. Thus, "nutraceutical compositions" according to the present invention can serve as supplements to food, feed and beverages, dietary supplements and as pharmaceutical formulations which may be solid - such as capsules or tablets - or liquid - such as solutions or suspensions. It is evident from the foregoing that the term "nutraceutical composition" also comprises food, feed and beverages containing EGCG.

In particular the present invention refers to the use of EGCG to increase
- the desire to exercise;
- the willingness to run and/or perform;
- the motivation to move and/or
- explorative behavior
of animals.

The terms "desire to exercise", "willingness to run and/or perform" and "motivation to move" as used herein include motivation for exercise, self-motivation (for exercise), willingness to perform endurance exercise as well as the urge to move.

According to the present invention the animals are preferably mammals, more preferably mammals selected from the group consisting of humans and pets (especially horses, dogs, cats and/or small animals, e.g. hamsters and/or guinea pigs). Most preferred are humans.

The EGCG may be administered in form of (fortified) food or (fortified) feed, dietary supplements, beverages, tablets, granules, capsules, pastes, gels, powders, food additives, feed additives, or effervescent formulations. Further examples are cereals, cereal bars, dairy products (e. g. milk, buttermilk, soured milk, yogurt (drinks), curd, quark desserts and so on) and pasta (especially tomato) sauces containing EGCG.

In a preferred embodiment of the present invention EGCG is administered on a regular (preferred daily) basis and/or in due time (e.g. several weeks) before the beginning of the exercise or before a competition, match, tournament and the like.

In preferred embodiments of the uses as given above the daily dosage of EGCG varies from 0.1 to 30 mg per kg body weight per day, preferably from 1.4 to 8.5 mg per kg body weight per day, more preferably from 2 to 4.5 mg per kg body weight per day, most preferably from 4.0 to 4.5 mg per kg body weight per day.

Thus, for humans with a weight of 70 kg, the daily dosage of EGCG varies from 7 to 2100 mg, preferably from 100 to 600 mg, more preferably from 140 to 300 mg; most preferably it is about 300 mg per day.

Skeletal muscles contain two main types of fibers, which differ in the mechanism they use to produce ATP; the amount of each type of fiber varies from muscle to muscle and from person to person.

Red ("slow-twitch") fibers have more mitochondria, store oxygen in myoglobin, rely on aerobic metabolism, have a greater capillary to volume ratio and are associated with endurance; these produce ATP more slowly. Marathon runners tend to have more red fibers, generally through a combination of genetics and training. "Slow-twitch" fibers are termed Type I fibers (see Table below). Therefore, the more Type I fibers an individual has the better its skeletal muscle can conduct endurance activities and the higher is its resistance to fatigue.

White ("fast-twitch") fibers have fewer mitochondria, are capable of more powerful (but shorter) contractions, metabolize ATP more quickly, have a lower capillary to volume ratio, and are more likely to accumulate lactic acid. Weightlifters and sprinters tend to have more white fibers. Three major fiber types are defined as "fast-twitch" fibers, which are Type IIa fibers, Type IId/x fibers and Type IIb fibers (Table). Among these fibers Type IIa fibers have a predominantly aerobic metabolism and are therefore utilized to support endurance activities. Type IIb fibers on the other hand have a predominantly glycolytic metabolism, are fatiguing quickly and are not suitable for long-term endurance activities. Therefore, if an individual has more Type IIa fibers its skeletal muscle has a higher capacity to conduct endurance activities whereas individuals with more abundant Type IIb fibers are likely to fatigue quicker during endurance activities.

**Table: Characteristics of muscle fiber types**

| **Fiber Type** | **Type I** | **Type IIa** | **Type IId/x** | **Type IIb** |
|---|---|---|---|---|
| Contraction time | Slow | Moderately fast | Fast | Very fast |
| Size of motor neuron | Small | Medium | Large | Very large |
| Resistance to fatigue | High | Fairly high | Intermediate | Low |
| Activity Used for | Aerobic | Long-term aerobic | Short-term anaerobic | Short-term anaerobic |
| Force production | Low | Medium | High | Very high |
| Mitochondrial density | High | High | Medium | Low |
| Capillary density | High | Intermediate | Low | Low |
| Oxidative capacity | High | High | Intermediate | Low |
| Glycolytic capacity | Low | High | High | High |
| | Triglycerides | Creatine phos-phate, glycogen | Creatine | Creatine |
| Major storage fuel | | | phosphate, | phosphate, |
| | | | glycogen | glycogen |

Accordingly a further object of the present invention is the use of EGCG to increase
- the number of oxidative ("slow-twitch") muscle fibers;
- the number of aerobic skeletal muscle fibers which can help to perform endurance exercise and/or
- fiber type shift towards aerobic skeletal muscle fibers
in animals.

According to the present invention the animals are preferably mammals, more preferably mammals selected from the group consisting of humans and pets (especially horses, dogs, cats and/or small animals, e.g. hamsters and/or guinea pigs). Most preferred are humans.

It was not to be forseen by the person skilled in the art that the consumption of EGCG would exert changes in fiber type composition of skeletal muscle similar to regular endurance exercise, even without any (endurance) exercise. It was especially surprising that the number of oxidative muscle fibers was increased independent from any physical exercise, i.e. in sedentary and trained individuals similarly.

The preferences mentioned above for the purity of EGCG, the forms in which it can be administered, and its daily dosage also apply here.

Further object of the present invention is a method for increasing
- the desire to exercise;
- the willingness to run and/or perform;
- the motivation to move and/or
- explorative behavior
of animals, said method comprising the step of administering an effective dose of EGCG to said animal, whereas the effective dose of EGCG varies from 0.1 to 30 mg per kg body weight per day, preferably from 1.4 to 8.5 mg per kg body weight per day, more preferably from 2 to 4.5 mg per kg body weight per day, most preferably from 4.0 to 4.5 mg per kg body weight per day.

Further object of the present invention is a method for increasing
- the number of oxidative ("slow-twitch") muscle fibers;
- the number of aerobic skeletal muscle fibers which can help to perform endurance exercise and/or
- fiber type shift towards aerobic skeletal muscle fibers
in animals, said method comprising the step of administering an effective dose of EGCG to said animal, whereas the effective dose of EGCG varies from 0.1 to 30 mg per kg body weight per day, preferably from 1.4 to 8.5 mg per kg body weight per day, more preferably from 2 to 4.5 mg per kg body weight per day, most preferably from 4.0 to 4.5 mg per kg body weight per day.

The preferences and explanations mentioned above also apply here.

A further object of the present invention is the use of EGCG for the manufacture of a composition for improving
- the desire to exercise;
- the willingness to run and/or perform;
- the motivation to move and/or
- explorative behavior
of animals.

A further object of the present invention is the use of EGCG for the manufacture of a composition for improving
- the number of oxidative ("slow-twitch") muscle fibers;
- the number of aerobic skeletal muscle fibers which can help to perform endurance exercise and/or
- fiber type shift towards aerobic skeletal muscle fibers
in animals.

The preferences and explanations mentioned above also apply here.

If the composition is prepared in form of tablets, capsules, granules or powder for oral administration, there may be used excipients such as lactose, sucrose, sodium chloride, glucose, urea, starch, dextrins and/or maltodextrins, calcium carbonate, calcium phosphate and/or calcium hydrogen phosphate, kaolin, crystalline and/or microcrystalline cellulose and/or silicic acid as carriers; binders such as water, ethanol, propanol, simple syrup, glucose solution, starch and/or hydrolyzed starch solution, gelatin solution, carboxymethylcellulose, hydroxypropylcellulose, hydroxypropylstarch, shellac, methylcellulose, ethylcellulose, calcium phosphate and/or polyvinyl pyrrolidone; disintegrators such as dry starch, croscarmellose, crospovidone, sodium alginate, agar powder, laminaran powder, sodium hydrogencarbonate, calcium carbonate, polyoxyethylene sorbitan fatty acid esters, sodium lauryl sulfate, stearic acid monoglyceride, starch and/or lactose; disintegration-preventing agents such as stearic acid, cacao butter and/or hydrogenated oils; absorbefacients such as quaternary ammonium bases and/or sodium lauryl sulfate; humectants such as glycerol and/or starch; adsorbents such as starch, lactose, kaolin, bentonite and/or colloidal silica; lubricants such as purified talc, stearic acid salts, boric acid powder and/or polyethylene glycol; taste corrigents such as sucrose, orange peel, citric acid and/or succinic acid; and the like.

If the composition is prepared in the form of tablets, these may be provided as tablets coated with usual coatings, for example, sugar-coated tablets, gelatin-coated tablets, enteric coated tablets, film-coated tablets, double coated tablets, multilayer-coated tablets and the like. The capsules are prepared by mixing the compounds according to the present invention with the various carriers exemplified above or according to the current state of the art and charging the mixture into hard gelatin capsules, soft capsules and the like.

A multi-vitamin and mineral supplement may be added to the compositions according to the present invention, e.g. to maintain a good balanced nutrition or to obtain an adequate amount of an essential nutrient missing in some diets. The multi-vitamin and mineral supplement may also be useful for disease prevention and protection against nutritional losses and deficiencies due to lifestyle patterns and common inadequate dietary patterns sometimes observed in diabetes.

The composition according to the present invention can be a food or beverage composition. Beverages can be e.g. sports drinks, energy drinks or other soft drinks, or any other suitable beverage preparation, e.g. joghurt drinks, hot beverages or soups.
In a preferred embodiment of the present invention the beverage is an "instant beverage", i.e. a beverage which is produced - normally by the consumer themself - by stirring a powder into a liquid, usually milk or water.

By "sports drink" a beverage is meant which is consumed before, during and/or after physical exercise, mainly to hydrate as well as to (re-) store electrolytes, sugar and other nutrients. Sports drinks are usually isotonic, meaning they contain the same proportions of nutrients as found in the human body.

Energy drinks are beverages which contain (legal) stimulants, vitamins (especially B vitamins) and/or minerals with the intent to give the user a burst of energy. Common ingredients include caffeine, guarana (caffeine from the Guarana plant) and/or taurine, various forms of ginseng, maltodextrin, inositol, carnitine, creatine, glucuronolactone, coenzyme Q10 and/or ginkgo biloba. Some may contain high levels of sugar, or glucose, whereas others are sweetened completely or partially with a sugar alcohol and/or an artificial sweetener like Cacyclamate or Aspartame. Many such beverages are flavored and/or colored.

A soft drink is a drink that does not contain alcohol. In general, the term is used only for cold beverages. Hot chocolate, tea, and coffee are not considered soft drinks. The term includes carbonated and non-carbonated drinks, e.g. mineral water or so-called "near water drinks", i.e. water-based beverages which have an additional benefit, e.g. a special flavor and/or further (functional) ingredients. One simple example for a "near water drink" is a mixture of water with very little juice.

If the composition is prepared in form of one of the following food articles it is according to the present invention advantageous if the amount of EGCG in the composition is selected from the ranges given in the following table:

| **Food Category** | **typical serving size** | **mg EGCG per L beverage or kg food** |
|---|---|---|
| Beverages (final product) (e.g. soft drinks, juices (fruit and/or vegetable), teas, soups) | 250 mL, preferred 3 servings a day | 20 to 600, preferred 100 to 400 |
| Dairy Products (e.g. milk shakes, joghurts, joghurt drinks, ice creams) | 150 g | 170 to 1'000 |
| Sweets and confectionaries (e.g. chocolates, candies, mints, jellies, cookies) | 1 to 5 pieces of 5 g each per day = 5 to 25 g | 500 to 10'000, preferred 500 to 1'000 |
| other food items (e.g. breakfast cereals, muesli bars, snacks, pasta sauces) | 25 g | 400 to 4'000, preferred 400 to 1'400 |

If the composition is prepared in form of a tablet or a capsule it is according to the present invention advantageous if the amount of EGCG is selected from the ranges given in the following table:

| **Category** | **typical dosage** | **mg EGCG per tablet/capsule** |
|---|---|---|
| Tablets | 1-2 x per day | 50 to 150 |
| Capsules | 1-2 x per day | 50 to 150 |

The invention is further illustrated by the following examples.

### Examples

### Example 1:

Male C57BL/6J mice (5 weeks old) were purchased from Charles River (Sulzfeld, Germany). All mice initially received a high-fat diet (Kliba#2154, Provimi Kliba AG, Kaiseraugst, Switzerland) for 3 months. Thereafter, mice were randomly assigned to 5 groups.
- Group 1: Low-fat diet, restricted to cage normal activity (Low fat sedentary, n=11)
- Group 2: High-fat diet, restricted to cage normal activity (High fat sedentary, n=11)
- Group 3: High-fat diet containing 0.5% w/w EGCG, restricted to cage normal activity (High fat + EGCG sedentary, n=10)
- Group 4: High-fat diet, free access to running wheel (High fat exercise, n=10)
- Group 5: High-fat diet containing 0.5% w/w EGCG, free access to running wheel (High fat + EGCG exercise, n=10)

The duration of the treatment was 2 months. During this period all mice received food and water *ad libitum.* Mice in groups 4 and 5 had unlimited access to running wheels (Technical & Scientific Equipment GmbH, Bad Homburg, Germany). Daily voluntary running wheel activity was monitored. At the end of the treatment period, maximal running performance on a rodent treadmill (Technical & Scientific Equipment GmbH, Bad Homburg, Germany) was determined. The Gastrocnemius muscles were excised, frozen in liquid nitrogen and stored at - 80°C for later quantification of the fiber type. In the medial gastrocnemius, skeletal muscle fibers were quantified as Type I, Type IIA, Type D/X, Type IIB by immune-histochemistry utilizing fiber specific antibodies and by histochemistry using a cytochrome oxidase staining.

The voluntary running wheel activity of mice receiving the high-fat diet containing EGCG was significantly increased compared to mice receiving the high-fat diet alone: Mice consuming EGCG containing high-fat diets ran on average 3140.3 ± 43.3 m per day while mice on the high fat diet ran only 1579.8 ± 59.0 m per day (P<0.004).

Supplementation of the high-fat diet with EGCG increased the percentages of Type I and Type IIa fibers in the medial gastrocnemius muscle of sedentary and exercised mice compared to sedentary mice receiving the high-fat diet alone (Table 1). Type IId/x and Type IIb fibers were less prevalent in the medial gastrocnemius muscle of mice receiving EGCG in the high-fat diet compared to mice receiving the high-fat diet alone (Table 1). The observed fiber type shift in both EGCG groups was more pronounced then the fiber type shift observed in the exercised high-fat group (Table 1). Exercise alone resulted in an increased proportion of Type IIa fibers (Table 1).

**Table 1: Percentage of fibers in medial gastrocnemius muscle of mice receiving the respective treatments for 2 months.**

| **Group** | **Type I** | **Type IIa** | **Type IId/x** | **Type IIb** |
|---|---|---|---|---|
| Low fat sedentary | 7.5 | 40.8 | 9.4 | 42.3 |
| High fat sedentary | 7.8 | 42.3 | 10.7 | 39.2 |
| High fat + EGCG sedentary | 12.5 | 53.5 | 5.2 | 28.8 |
| High fat exercise | 6.3 | 49.8 | 5.7 | 38.1 |
| High fat + EGCG exercise | 12.8 | 54.2 | 5.1 | 27.8 |

### Example 2: Pharmaceutical compositions

### Soft gelatine capsule:

The soft gelatine capsule is prepared by conventional formulation procedures using the ingredients specified below:
Active ingredient: EGCG 150 mg and vitamin E 50 mg
Other ingredients: glycerol, water, gelatine, vegetable oil.

### Hard gelatine capsule:

The hard gelatine capsule is prepared by conventional formulation procedures using the ingredients specified below:
Active ingredient: EGCG 300 mg
Other ingredients:
   Fillers: lactose, cellulose and/or cellulose derivatives.
   Lubricant: magnesium stearate if necessary (0.5 % by weight)

### Tablet:

The tablet is prepared by conventional formulation procedures using the ingredients specified below:
Active ingredient: EGCG 300 mg and vitamin E 20 mg
Other ingredients: microcrystalline cellulose, silicone dioxide, magnesium stearate, crosscarmellose sodium.

### Example 3: Food items

### Instant drink:

### Composition:

| | **Parts** |
|---|---|
| Xylitol | 669.4 |
| Citric acid, anhydrous | 220.0 |
| Carboxymethyl cellulose | 30.0 |
| Tri-sodium-citrate | 22.0 |
| Tri-calcium-phosphate | 20.0 |
| Orange flavour | 20.0 |
| Ascorbic acid | 8.0 |
| Sweetener Twinsweet | 4.0 |
| EGCG as TEAVIGO^{™} | 6.6 |
| Powder mix total | 1000.0 |

### Preparation:

Sieve all ingredients through a 500 µm sieve. Give the powder in an appropriate container and mix on a tumbler blender for at least 20 min. Use 35 g powder for one litre of beverage by adding water.

The instant drink contains 50 mg EGCG per serving of 240 ml ready drink.

Up to 3 servings per day is recommended.

### Mints:

### Composition:

| | Potency/mint | Amount/min t |
|---|---|---|
| EGCG (as TEAVIGO^{™}TG) | 10 mg | 11.1 mg |
| Vitamin C as Ascorbic Acid fine granular | | 10.5 mg |
| Sorbitol as Neosorb 60 W | | 163.6 mg |
| Silicon dioxide (Aerosil ^{™} 200) | | 1 mg |
| Aroma Frescoforte Permaseal 60470-31 (Givaudan) | | 10 mg |
| Aroma Eiszucker Permaseal 60153-73 (Givaudan) | | 6.0 mg |
| Sweetener as Twinsweet | | 1.6 mg |
| PEG 6000 | | 20.0 mg |
| Magnesium stearate | | 1.2 mg |
| Total Weight | | 225.0 mg |

### Preparation:

Mix TEAVIGO^{™} TG, Ascorbic Acid fine granular, Sorbitol, the aromas, sweetener and PEG 6000 into a drum and mix for 10 minutes with a tumbler mixer. Sieve Sorbitol and silicon dioxide through a 1 mm sieve and mix it in a separate drum for 10 minutes. Combine the two powder mixtures and mix again for 10 minutes. Add Magnesium stearate after sieving through a 1 mm sieve and mix for 2 minutes.
TEAVIGO^{™} , TEAVIGO^{™} TG: Tradeproducts of DSM Nutritional Products.

The powder is compressed to tablets with a Korsch XP1 tablet press, punch size 8 mm round.
Up to 5 mints per day are recommended.

### Soft Drink (Near Water):

Typical serving: 250 ml
Active ingredient: EGCG: 5-500 mg/per serving

## Claims

1. Use of (-)-epigallocatechin gallate and/or one or more of its derivatives to increase
• the desire to exercise;
• the willingness to run and/or perform;
• the motivation to move and/or
• explorative behavior
of animals.

2. Use of (-)-epigallocatechin gallate and/or one or more of its derivatives to increase
• the number of oxidative ("slow-twitch") muscle fibers;
• the number of aerobic skeletal muscle fibers which can help to perform endurance exercise and/or
• fiber type shift towards aerobic skeletal muscle fibers
in animals.

3. Method for increasing
• the desire to exercise;
• the willingness to run and/or perform;
• the motivation to move and/or
• explorative behavior
of animals, said method comprising the step of administering an effective dose of EGCG to said animal, whereas the effective dose of EGCG varies from 0.1 to 30 mg per kg body weight per day.

4. Method for increasing
• the number of oxidative ("slow-twitch") muscle fibers;
• the number of aerobic skeletal muscle fibers which can help to perform endurance exercise and/or
• fiber type shift towards aerobic skeletal muscle fibers
in animals, said method comprising the step of administering an effective dose of EGCG to said animal, whereas the effective dose of EGCG varies from 0.1 to 30 mg per kg body weight per day.

5. Method according to any of the claims 3 or 4, wherein the effective dose of EGCG varies from from 2 to 4.5 mg per kg body weight per day.

6. Use of (-)-epigallocatechin gallate and/or one or more of its derivatives for the manufacture of a composition for increasing
• the desire to exercise;
• the willingness to run and/or perform;
• the motivation to move and/or
• explorative behavior
of animals.

7. Use of (-)-epigallocatechin gallate and/or one or more of its derivatives for the manufacture of a composition for increasing
• the number of oxidative ("slow-twitch") muscle fibers;
• the number of aerobic skeletal muscle fibers which can help to perform endurance exercise and/or
• fiber type shift towards aerobic skeletal muscle fibers
in animals.

8. Use or method according to any of the preceding claims, wherein (-)-epigallocatechin gallate itself is used.

9. Use or method according to any of the preceding claims, wherein the animal is a mammal.

10. Use or method according to claim 9, wherein the mammal is selected from the group consisting of humans and pets.
